(19) Europäisches Patentamt
European Patent Office
Office européen des brevets

(11) Numéro de publication : **0 445 050 B1**

# (12) FASCICULE DE BREVET EUROPEEN

(45) Date de publication du fascicule du brevet : **27.04.94 Bulletin 94/17**

(51) Int. Cl.$^5$ : **C07C 39/16,** C07C 37/20

(21) Numéro de dépôt : **91420065.4**

(22) Date de dépôt : **25.02.91**

## (54) Procédé de fabrication du bisphénol-A.

(30) Priorité : **28.02.90 FR 9002758**

(43) Date de publication de la demande : **04.09.91 Bulletin 91/36**

(45) Mention de la délivrance du brevet : **27.04.94 Bulletin 94/17**

(84) Etats contractants désignés : **BE CH DE ES FR GB GR IT LI NL**

(56) Documents cités :
**DE-A- 1 618 016**
**US-A- 4 052 466**
**US-A- 4 317 944**
**US-A- 4 387 251**

(73) Titulaire : **RHONE POULENC CHIMIE**
**25 Quai Paul Doumer**
**F-92408 Courbevoie Cédex (FR)**

(72) Inventeur : **Desmurs, Jean-Roger**
**La Jonquière - Route de Ternay**
**F-69360 Saint-Symphorien D'Ozon (FR)**
Inventeur : **Pierre, Francis**
**30,rue Louis Jouvet Les Hobrelles IV**
**F-69740 Genas (FR)**

(74) Mandataire : **Dubruc, Philippe et al**
**RHONE-POULENC CHIMIE Direction de la Propriété Industrielle 25, Quai Paul Doumer**
**F-92408 Courbevoie Cedex (FR)**

EP 0 445 050 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention a pour objet un procédé de préparation du bisphénol-A par condensation du phénol et de l'acétone.

Elle a plus particulièrement trait à un procédé de préparation du bisphénol-A par condensation du phénol et de l'acétone en présence d'un catalyseur acide et d'un additif ou co-catalyseur.

Le bisphénol-A ou bis(hydroxy-4 phényl)-2,2 propane est un intermédiaire utile dans la fabrication des résines époxy et polycarbonates.

De nombreux procédés de fabrication du bisphénol-A sont connus. Le plus répandu comprend la mise en réaction de l'acétone avec un excès de phénol en présence d'un catalyseur acide à des températures de l'ordre de 30 à 80°C.

Le catalyseur peut être un acide minéral, solide ou liquide, tel une argile, le trichlorure d'aluminium, l'acide chlorhydrique ou sulfurique ou un acide organique tel l'acide benzène sulfonique (cf. la demande japonaise 62-5926 publiée en 1962 et le brevet américain n° 4 387 251) ou une résine échangeuse d'ions (cf. AIChEJ. 1974, 20 (5) pp 933-40).

Les procédés mettant en oeuvre des catalyseurs hétérogènes présentent l'inconvénient majeur d'obliger à travailler à faible concentration de bisphénol-A, de manière à éviter la précipitation du complexe (bisphénol-A-phénol), ou à plus haute température, ce qui favorise la formation de sous produits. C'est pourquoi on recourt encore préférentiellement à un acide soluble dans le milieu de réaction tel l'acide chlorhydrique. L'acide chlorhydrique, quant à lui, présente l'inconvénient majeur de nécessiter des réacteurs en matériaux capables de résister à de fortes corrosions et donc très coûteux. La majorité des autres acides étant moins actifs que l'acide chlorhydrique, on est conduit à augmenter les temps de contact pour obtenir un taux de transformation acceptable voire complet de l'acétone. Or, l'augmentation du temps de contact se traduit par l'apparition d'espèces parasites particulièrement indésirables telles l'(o-hydroxyphényl)-2-(p-hydroxyphényl)-2 propane (isomère o,p' du bisphénol-A), le triméthyl-2,2,4 (p-hydroxyphényl)-4 chromane ou codimère, et des produits de condensation supérieure tel le trisphénol. Ces espèces ne peuvent être séparées du bisphénol-A que par une succession d'étapes de purification souvent délicates à mettre en oeuvre et toujours relativement onéreuses.

Par ailleurs pour certaines applications, il est impératif que le bisphénol-A n'ait aucune coloration et possède un très haut degré de pureté. Tel est le cas, notamment, de l'utilisation du bisphénol-A dans la synthèse des résines polycarbonates, dans laquelle la présence de substances étrangères a un effet inhibiteur marqué.

Dans ce contexte il a déjà été proposé de joindre au système (phénol/acétone/catalyseur acide) un "co-catalyseur" ou additif.

Le plus souvent le co-catalyseur est un composé soufré tel un alkyl-mercaptan (cf. la demande de brevet néerlandaise n° 73/09229), un acide alkyl-mercapto-carboxylique ou sulfonique (cf. le brevet australien n° 474 155 et le brevet français n° 1 179 377), ou un amino-alcanethiol (cf. la demande de brevet japonais n° 74/20 565).

Toutefois, l'emploi de ces accélérateurs ne se revèle pas pleinement avantageux : ils conduisent encore à de sérieuses difficultés de traitement pour les éliminer et/ou les récupérer et ils confèrent bien souvent au produit final une odeur non souhaitée.

L'utilisation d'une autre classe de "co-catalyseurs" a été préconisée dans le brevet américain n° 4 052 466, à savoir des dérivés poly(hydroxy)benzéniques, en particulier la résorcine et ses éthers mono-ou diméthyliques, que l'on utilise à raison de 0,1 à 10 % molaire par rapport au phénol engagé. Ces dérivés conduisent à des résultats similaires à ceux obtenus avec, par exemple, les mercaptans. Ils activent en effet la condensation phénol/acétone en présence d'un catalyseur acide et tendent à diminuer la chimie parasite.

Toutefois ces dérivés présentent l'inconvénient majeur de suivre le même chemin que les impuretés organiques du bisphénol-A dans la chaîne de production. Ils influent négativement sur la cristallisation de l'adduct bisphénol-A-phénol et restent présents au moins à titre de traces dans le produit final.

Aussi, dans un tel contexte est-il aisé de concevoir qu'il serait souhaitable de disposer d'additifs capables d'activer la réaction de condensation du phénol et de l'acétone, sans favoriser une chimie parasite, et qui suivraient un cheminement différent de celui des impuretés et/ou du bisphénol-A dans la chaîne de production.

La présente invention a donc pour objet un procédé de préparation du bisphénol-A par condensation du phénol en excès sur l'acétone en présence d'un catalyseur acide et d'un additif caractérisé en ce que l'additif répond à la formule (I)

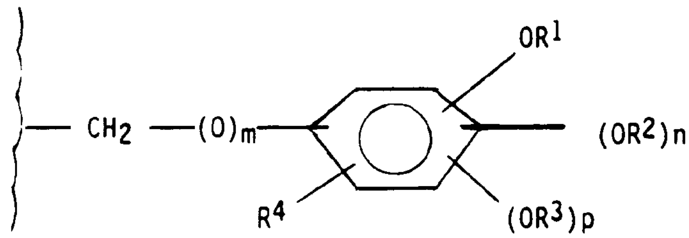 (I)

dans laquelle :

- m vaut 0 ou 1 ;
- n vaut 0 ou 1 ;
- p vaut 0 ou 1 ;
- $R^1$, $R^2$ et $R^3$, identiques ou différents, représentent un atome d'hydrogène, un radical alkyle en $C_1$-$C_6$ ou un radical phényle éventuellement substitué par 1 ou 2 groupements hydroxy ou alcoxy en $C_1$-$C_4$ ;
- $R^4$ représente un atome d'hydrogène, un atome d'halogène, un radical alkyle en $C_1$-$C_6$ ou l'un des groupements suivants :
  - CHO, - $NO_2$, - $CO_2H$, - $CO_2R^5$ où $R^5$ est un radical alkyle en $C_1$-$C_4$,

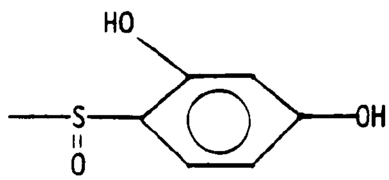 ;

et,

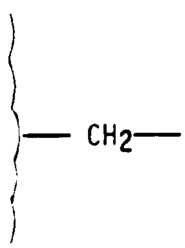

représente un reste de résine chlorométhylée.

Lorsque $R^4$ représente un atome d'halogène il est de préférence choisi parmi le chlore et le brome.

La résine chlorométhylée dont dérive l'additif est du type "polystyrène-divinylbenzène" dans lequel le taux de divinylbenzène est supérieur ou égal à 6 %. A titre d'exemples de telles résines convenant à la mise en oeuvre du présent procédé on peut citer : les résines BIO-BEAD® S-X1 de la Société Biorad, les résines DUOLITE® LES 9001 et DUOLITE® LES 3781 fournies par la Société ROHM et HAAS.

Les résines se présentant sous forme de perles conviennent plus particulièrement à la mise en oeuvre du présent procédé.

Pour une bonne mise en oeuvre de la présente invention, au moins 90 % des atomes de chlore de la résine chlorométhylée seront remplacés par un groupe (ou greffon) de formule II :

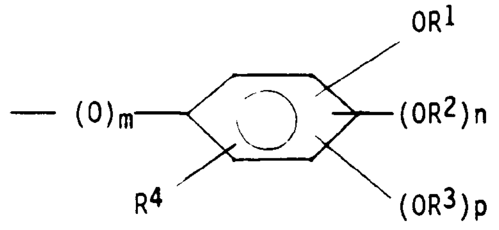

(II)

dans laquelle n, m, p, et $R^1$ à $R^4$ ont la signification donnée précédemment.

Les additifs en cause peuvent être préparés par transposition de l'une ou l'autre des méthodes décrites respectivement dans

(1) J. of Polymer Science - Part A, vol. 3 - 1965 - p. 1833-43 (K.A. KUN)

(2) Thèse de Doctorat d'Etat du 21.10.1981 - Université P & M. CURIE - Paris 6e (T.D. NGUYEN).

Selon la méthode décrite par (1) on obtiendra des additifs de formule (I) dans laquelle m vaut 0 ; selon la méthode décrite par (2) on obtiendra des additifs répondant à la formule (I) dans laquelle m vaut 1 ; les additifs pour lesquels m vaut 0 étant préférés.

Brièvement la méthode décrite par (1) comprend la réaction du diphénol sur la résine chlorométhylée, dans le dioxanne sec, au reflux, en présence de chlorure de zinc anhydre ; la méthode décrite par (2) comprend la réaction du phénate correspondant sur la résine chlorométhylée dans des conditions de catalyse par transfert de phase, à savoir dans le diméthylformamide, à 60°C en présence de soude aqueuse et d'hydrogénosulfate de tétrabutylammonium.

Les groupes de formule (II) ci-avant résultent de l'enlèvement d'un atome d'hydrogène à un composé aromatique di- ou polysubstitué.

A titre d'exemple de tels composés on peu citer : la résorcine, le pyrrogallol, la bromo-2 résorcine, l'acide dihydroxy-2,6 benzoïque et son ester méthylique, le dihydroxy-2,4 benzaldéhyde, le bis(dihydroxy-2,4 phényl) sulfoxyde, le monométhyléther de la résorcine et la n-hexyl-4 résorcine.

De préférence, on recourt à des additifs répondant à la formule (I) dans laquelle :

- m est nul ;
- $R^4$ représente un atome d'hydrogène ou un radical alkyle en $C_1$-$C_6$ ;
- $R^1$, $R^2$ et $R^3$, identiques ou différents, représentent un atome d'hydrogène ou un radical alkyle en $C_1$-$C_4$.

Pour une bonne mise en oeuvre de la présente invention, les dits additifs répondent à la formule (I) dans laquelle l'un au moins des radicaux $R^1$ à $R^3$ représente un atome d'hydrogène et les deux autres, identiques ou différents, représentent un atome d'hydrogène ou un radical méthyle.

Des additifs particulièrement avantageux répondent à la formule (I) dans laquelle :

- m est nul ;
- p est nul ;
- $R^4$ représente un atome d'hydrogène ou un radical alkyle en $C_1$-$C_6$ ;
- les deux radicaux $R^1$ et $R^2$ représentent un atome d'hydrogène, les groupements hydroxyles ainsi présents sont en positions 2, 4 sur le noyau aromatique par rapport au groupement méthylène.

La quantité d'additif à mettre en oeuvre n'est pas critique. En général, on utilise une quantité d'additif telle que le rapport molaire des groupes de formule (II) ci-avant à l'acétone soit compris entre 0,1 et 3 et, de préférence, entre 0,5 et 1,5.

Comme rappelé en tête du présent mémoire la réaction de condensation du phénol et de l'acétone est réalisée en présence d'un catalyseur acide. N'importe quel type d'acide, sous réserve bien entendu qu'il ne dégrade pas la résine, constituant du co-catalyseur, et qu'il ne s'introduise pas sur le noyau aromatique du greffon, est susceptible de convenir à la mise en oeuvre du présent procédé.

En effet, on peu recourir à des acides minéraux tel $HClO_4$ ou organiques tels l'acide méthane-sulfonique, l'acide benzène sulfonique et les résines porteuses de groupements acides sulfoniques.

L'acide benzène sulfonique convient bien à la mise en oeuvre du présent procédé.

La quantité d'acide est en général d'au moins 0.1 mole par mole d'acétone ; elle est de préférence comprise entre 0,2 et 0,5 mole par mole d'acétone.

La réaction est conduite avec un excès de phénol. Le rapport molaire phénol/acétone est généralement compris entre 2 et 16 et, de préférence entre 8 et 12.

La condensation du phénol et de l'acétone est conduite à une température supérieure à 30°C pour obtenir une activité raisonnable et inférieure à 120°C, température au delà de laquelle l'additif est susceptible de se dégrader et une chimie parasite est susceptible de perturber la qualité de la condensation recherchée.

Pour une bonne mise en oeuvre du procédé selon l'invention la Demanderesse préconise une température comprise entre 30 et 80°C.

En fin de réaction ou du temps imparti à celle-ci, on filtre le mélange réactionnel, on récupère alors le cocatalyseur qui peut-être réengagé à une nouvelle opération de condensation, après son lavage au phénol. Le filtrat, renfermant le bisphénol-A, le phénol et, le cas échéant, l'acétone qui n'a pas réagi, est soumis de manière en soi connue à des traitements (distillation, flash...) en vue de récupérer le bisphénol-A.

Les exemples ci-après illustrent la présente invention.

EXEMPLES 1 A 3 : Conditionnement des résines chlorométhylées

Un volume de 50 ml de résine est placé dans une colonne à chromatographier ; on laisse passer lentement 500 ml de méthanol répartis en 3 portions de respectivement 100 ml, 100 ml et 300 ml. La résine est ensuite séchée à l'étuve à, 70°C sous vide (environ 100 torr) pendant 12 heures.

Selon le mode opératoire décrit ci-avant on traite les 3 échantillons suivants :

EXEMPLE 1 : DUOLITE® LES 9001, résine chlorométhylée fournie par la Société ROHM et HAAS.
EXEMPLE 2 : DUOLITE® LES 3781, résine chlorométhylée fournie par la Société ROHM et HAAS.
EXEMPLE 3 : BIO-BEADS® S-X1, résine chlorométhylée commercialisée par la Société BIORAD.

Les analyses en eau et en chlore de ces résines conduisent aux résultats suivants :

| | Résine support | | |
|---|---|---|---|
| | 1<br>Duolite LES-9001 | 2<br>Duolite LES-3781 | 3<br>Bio-Beads S-X1 |
| % $H_2O$<br>(Karl-Fischer) | 0,002 | 0,06 | 0,057 |
| % Cl<br>(meq. Cl/g) | 21,77<br>(6,14) | 20,21<br>(5,70) | 14,89<br>(4,20) |
| granulométrie | 18-35 mesh<br>(500-1000 µm) | 18-35 mesh<br>(500-1000 µm) | 200-400 mesh<br>(37-74 µm) |

EXEMPLE 4 : C-Alkylation de la résine Duolite LES-9001 par la résorcine

Dans un ballon de 250 ml équipé d'un réfrigérant, d'un thermomètre, d'une arrivée d'argon et d'une agitation magnétique, on charge 10 g de résine Duolite LES-9001 (Traitée dans l'exemple 1) (61,4 mEq de $Cl^-$), 70 ml de dioxanne séché sur tamis 5 Å et 10,14 g de résorcine (91,1 mmol). Après dix minutes d'agitation, on ajoute 0,40 g de chlorure de zinc fraîchement fondu (2,93 mmol). Le mélange réactionnel est porté à reflux durant 24 heures. Après refroidissement à 70°C, la résine est filtrée puis lavée à température ambiante avec 2 x 100 ml de dioxanne puis 3 x 50 ml d'éthanol. La résine est ensuite purifiée par extraction au méthanol pendant 24 heures et est séchée sous vide (100 torr) à 45-50°C. On recueille 11,39 g de résine dont les analyses conduisent aux résultats ci-après :

| | DUOLITE LES 9001 |
|---|---|
| % $H_2O$ | 1,65 |
| % Cl | 0,35 |
| % O | 11,19 |
| Granulométrie | 18-35 mesh<br>(500-1000 µm) |

Ceci correspond à une teneur de 4,1 mMol de résorcine par gramme de résine.

EXEMPLE 5 : C-Alkylation de la résine BIO-BEADS S-X1 par la résorcine.

On opère comme dans l'exemple 4 en chargeant :

- 8,25 g de la résine BIO-BEADS S-X1 (traitée dans l'exemple 3)
- 5,72 g de résorcine
- 0,23 g de chlorure de zinc fraichement fondu
- 80 ml de dioxanne sur tamis 5 Å

On recueille 9,4 g de résine dont les analyses conduisent aux résultats suivants :

| | RESINE BIO-BEADS S-X1 |
|---|---|
| % $H_2O$ | 0,5 |
| % Cl | 0,27 |
| % O | 6,67 |
| Granulométrie | 150-290 mesh (49-97 µm) |

Ces analyses correspondent à une teneur de 3,13 mMol de résorcine par gramme de résine.

EXEMPLE 6 : C-Alkylation de la résine DUOLITE LES 3781 par la résorcine

On opère comme dans l'exemple 4 en chargeant :

- 10 g de résine DUOLITE LES 3781 (traitée dans l'exemple 2)
- 9,41 g de résorcine
- 0,37 g de chlorure de zinc fraîchement fondu
- 65 ml de dioxanne

On recueille 10,25 g de résine dont les analyses conduisent aux résultats suivants :

| | RESINE DUOLITE LES 3781 |
|---|---|
| % $H_2O$ | 1,2 |
| % Cl | 2 |
| % O | 8,52 |
| Granulométrie | 18-35 mesh (500-1000 µm) |

Ces analyses correspondent à une teneur de 3,57 mMol de résorcine par gramme de résine.

Essai témoin (a) : C-Alkylation de la résine DUOLITE LES 9001 par du phénol

On opère comme dans l'exemple 4 en chargeant

- 10 g de résine DUOLITE LES 9001 (traitée dans l'exemple 1)
- 8,67 g de phénol
- 0,40 g de chlorure de zinc fraîchement fondu

- 70 ml de dioxanne séché au tamis

On recueille 10,8 g de résine dont les analyses conduisent aux résultats suivants :

| | RESINE DUOLITE LES 9001 |
|---|---|
| % $H_2O$ | 1,02 |
| % Cl | 1,04 |
| % O | 5,91 |
| Granulométrie | 18-35 mesh (500-1000 μm) |

Ces analyses correspondent à une teneur en phénol de 4,27 mMol de phénol par g de résine.

EXEMPLE 7 : O-Alkylation de la résine DUOLITE LES 9001.

Dans un ballon de 250 ml équipé d'un réfrigérant, d'un thermomètre, d'une arrivée d'argon, d'une agitation magnétique, on charge 5 g de résine DUOLITE LES 9001 (traitée dans l'exemple 1) et 150 ml de DMF. Après avoir fait gonfler la résine pendant 42 heures à température ambiante, on additionne 0,51 g d'hydrogénosulfate de tétrabutylammonium, 4 ml de soude aqueuse à 50 % et 6,67 g de résorcine. Le mélange réactionnel est chauffé six heures à 65°C. La résine est ensuite filtrée, lavée avec 5 x 250 ml d'eau, 3 x 250 ml de méthanol, puis séchée.

On recueille 5,03 g de résine dont la analyses conduisent aux résultats suivants :

| | DUOLITE O-ALKYLEE |
|---|---|
| % $H_2O$ | 3,5 |
| % Cl | 4,38 |
| % O | 6,2 |
| Granulométrie | 18-35 mesh (500-1000 μm) |

Ces analyses correspondent à une teneur 2,54 mmol de résorcine par gramme de résine.

Essai témoin (b) : Synthèse du bisphénol-A catalysée par l'acide benzènesulfonique.

Dans un réacteur de 30 ml muni d'un agitateur magnétique, on charge 7,56 g de phénol (0,08 mole) et 0,56 g d'acide benzène sulfonique (0,0035 mole). Ce mélange est chauffé à 50°C. Lorsque la température du réacteur s'est stabilisée à 50°C, on introduit très rapidement à l'aide d'une seringue 0,58 g d'acétone (0.01 mole). Des prélèvements effectués à différents intervalles de temps sont dosés en chromatographie liquide à haute performance pour déterminer les rendements en bisphénol-A et en impuretés.

Les résultat obtenus sont consignés dans le tableau I ci-après dans lequel les conventions suivantes sont utilisées.

On a indiqué les rendements par rapport à l'acétone engagée pour les différents produits comme suit :

pp' pour l'isomère p,p' du bisphénol-A

op' pour l'isomère o,p' du bisphénol-A

BPX pour le trisphénol
Codimère pour le triméthyl-2,2,4 (p-hydroxyphényl)-4 chromane
Spiro pour le dihydroxy-6,6' tétraméthyl-3,3,3',3' spirobiindane-1,1'
ΣRR représente la somme des rendements obtenus pour les isomères o, p' et p, p' du bisphénol-A
Q représente la proportion d'isomère o, p' dans le mélange d'isomères o, p' et p,p' du bisphénol-A

TABLEAU I

---------

| Durée (h) | p p' | o p' | BPX | Codimère | Spiro | ΣRR | Q |
|---|---|---|---|---|---|---|---|
| 0,25 | 1,17 | 0,58 | - | - | - | 1,75 | 0,333 |
| 0,50 | 2,97 | 1,32 | - | - | - | 4,29 | 0,307 |
| 1,00 | 6,10 | 2,64 | 0,06 | 0,26 | - | 8,74 | 0,302 |
| 2,00 | 11,90 | 4,59 | 0,31 | 0,71 | - | 16,49 | 0,279 |
| 3,58 | 19,10 | 6,56 | 0,52 | 1,74 | 0,10 | 25,66 | 0,255 |
| 4,67 | 23,63 | 7,47 | 0,78 | 2,13 | 0,14 | 31,10 | 0,240 |
| 5,50 | 26,74 | 7,91 | 0,82 | 2,45 | 0,11 | 34,65 | 0,228 |
| 6,67 | 29,61 | 8,45 | 1,00 | 2,84 | 0,25 | 38,06 | 0,222 |

EXEMPLE 8 : Synthèse du bisphénol-A catalysée par l'acide benzènesulfonique en présence de résine DUOLITE LES 9001 C-alkylée par de la résorcine

Dans un réacteur de 30 ml muni d'un agitateur magnétique, on charge 7,56 g de phénol (0,08 mole) 0,56 g d'acide benzènesulfonique (0,0035 mole), 2,5 g de la résine préparée selon l'exemple 4 (0,01 mole de résorcine). Le mélange réactionnel est chauffé à 50°. Lorsque la température du mélange réactionnel a atteint 50°C, on introduit très rapidement à l'aide d'une seringue 0,58 g d'acétone (0,01 mole). Des prélèvements effectués à différents intervalles de temps sont dosés en chromatographie liquide à haute performance pour déterminer les rendements en bisphénol-A et impuretés.

Les résultats obtenus sont consignés dans le tableau II ci-après dans lequel les conventions utilisées sont celles définies précédemment pour l'essai témoin b.

TABLEAU II

----------

| Durée (h) | p p' | o p' | BPX | Codimère | Spiro | ΣRR | Q |
|---|---|---|---|---|---|---|---|
| 0,084 | 0,53 | 0,21 | 0,01 | - | - | 0,74 | 0,282 |
| 0,25 | 4,7 | 0,92 | 0,11 | - | - | 5,62 | 0,163 |
| 0,50 | 9,2 | 1,50 | 0,10 | - | - | 10,70 | 0,142 |
| 1,17 | 19,84 | 2,44 | 0,25 | 0,07 | - | 22,30 | 0,111 |
| 2,17 | 32,54 | 3,74 | 0,70 | 0,21 | 0,07 | 36,29 | 0,103 |
| 4,33 | 50,32 | 5,27 | 1,43 | 0,36 | 0,10 | 55,60 | 0,095 |
| 5,42 | 64,05 | 5,71 | 1,55 | 0,37 | 0,15 | 69,76 | 0,082 |
| 8,18 | 90,38 | 5,83 | 1,42 | 0,74 | 0,17 | 96,21 | 0,060 |

EXEMPLE 9 : Synthèse du bisphénol-A catalysées par l'acide benzènesulfonique en présence de résine BIO-BEADS S-X1 C-alkylée par de la résorcine

On opère comme dans l'exemple 8 en utilisant les réactifs suivants :

- 7,56 g de phénol (0,08 mole)
- 0,58 g d'acétone (0,01 mole)
- 0,56 g d'acide benzènesulfonique (0,0035 mole)
- 3,2 g de la résine préparée selon l'exemple 5 (0,01 mole de résorcine)

L'analyse des différents prélèvements montre une cinétique beaucoup plus rapide.

Les résultats obtenus sont consignés dans le tableau III ci-aprés dans lequel les conventions utilisées sont celles définies précédemment pour l'essai témoin b.

TABLEAU III

-----------

| Durée (h) | p p' | o p' | BPX | Codimère | Spiro | ΣRR | Q |
|---|---|---|---|---|---|---|---|
| 0,084 | 7,10 | 0,76 | 0,11 | - | - | 7,86 | 0,096 |
| 0,25 | 15,06 | 1,27 | 0,15 | - | - | 16,33 | 0,078 |
| 0,62 | 30,76 | 2,27 | 0,46 | - | - | 33,03 | 0,069 |
| 1,25 | 46,53 | 3,38 | 1,06 | 0,07 | 0,12 | 49,91 | 0,068 |
| 4,42 | 71,03 | 4,46 | 1,48 | 0,10 | 0,14 | 75,49 | 0,05 |

EXEMPLE 10 : Synthèse du bisphénol A catalysée par l'acide benzènesulfonique en présence de résine DUOLITE LES 3781 C-alkylés par la résorcine.

On opère comme dans l'exemple 8 en utilisant les réactifs suivants :
- 7,56 g du phénol (0,08 mole)
- 0,58 g d'acétone (0,01 mole)
- 0,56 g d'acide benzènesulfonique (0,0035 mole)
- 1,63 g de résine préparée selon l'exemple 6 (0,01 mole de résorcine)

Les analyses des prélèvements montrent un gain sensible sur la cinétique de la réaction.

Les résultats obtenus sont consignés dans le tableau IV ci-après dans lequel les conventions utilisées sont celles définies précédemment pour l'essai témoin b.

TABLEAU IV

----------

| Durée (h) | p p' | o p' | BPX | Codi-mère | Spiro | ΣRR | Q |
|---|---|---|---|---|---|---|---|
| 0,25 | 0,74 | 0,65 | 0,04 | - | - | 1,39 | 0,468 |
| 0,50 | 3,03 | 1,39 | 0,09 | 0,09 | - | 4,42 | 0,314 |
| 1,00 | 7,81 | 3,34 | 0,16 | 0,28 | - | 11,15 | 0,299 |
| 2,00 | 17,64 | 6,15 | 0,59 | 0,87 | 0,02 | 23,79 | 0,258 |
| 3,42 | 31,00 | 8,15 | 1,24 | 0,95 | 0,07 | 39,15 | 0,208 |
| 4,50 | 43,78 | 8,47 | 1,74 | 1,36 | 0,11 | 52,25 | 0,162 |
| 5,83 | 66,92 | 7,76 | 1,08 | 1,52 | 0,22 | 74,68 | 0,104 |
| 7,34 | 86,65 | 4,91 | 0,62 | 2,04 | 0,25 | 91,56 | 0,054 |

Essai témoin (c) : Synthèse du bisphénol-A catalysée par l'acide benzènesulfonique en présence de résine DUOLITE LES 9001 C-alkylée par du phénol.

On opère comme dans l'exemple 8 en chargeant les réactifs suivants :
- 7,56 g de phénol (0,08 mole)
- 0,58 g d'acétone (0,01 mole)
- 0,56 g d'acide benzènesulfonique (0,0035 mole)
- 2,34 g de résine préparée selon l'essai témoin (a) (0,01 mole de phénol)

Les analyses des prélèvements ne montrent aucun effet par rapport à l'essai témoin (b).

Les résultats obtenus sont consignés dans le tableau V ci-après dans lequel les conventions utilisées sont celles définies précédemment pour l'essai témoin b.

TABLEAU V

---------

| Durée (h) | p p' | o p' | BPX | Codimère | Spiro | ΣRR | Q |
|---|---|---|---|---|---|---|---|
| 0,84 | 2,80 | 1,36 | 0,08 | - | - | 4,16 | 0,327 |
| 1,84 | 7,16 | 2,79 | 0,12 | 0,22 | - | 9,95 | 0,280 |
| 3,50 | 15,11 | 4,04 | 0,41 | 1,10 | - | 19,15 | 0,211 |
| 4,42 | 18,54 | 4,67 | 0,56 | 1,43 | - | 23,21 | 0,201 |
| 5,42 | 22,36 | 6,51 | 0,80 | 2,18 | - | 28,87 | 0,225 |
| 6,67 | 28,95 | 7,38 | 0,85 | 2,27 | - | 36,33 | 0,203 |

Exemple 11 : Synthèse du bisphénol-A catalysée par l'acide benzènesulfonique en recyclant la résine DUOLITE LES 9001 C-alkylée par de la résorcine.

Un premier essai est réalisé dans les conditions de l'exemple 8, mais sans prélèvements. En fin d'essai (5 heures), la résine est filtrée, lavée au phénol et séchée puis engagée avec les réactifs suivants dans un second essai réalisé selon l'exemple 8 :

- 7,56 g de phénol (0,08 mole)
- 0,58 g d'acétone (0,01 mole)
- 0,56 g d'acide benzènesulfonique (0,0035 mole)
- 2,55 g de résine recyclée

L'analyse des prélèvements montrent que la résine présente toujours une activité.

Les résultats obtenus sont consignés dans le tableau VI ci-après dans lequel les conventions utilisées sont celles définies précédemment pour l'essai témoin b.

TABLEAU VI

---------

| Durée (h) | p p' | o p' | BPX | Codimère | Spiro | ΣRR | Q |
|---|---|---|---|---|---|---|---|
| 0,25 | 0,99 | 0,28 | 0,06 | - | - | 1,27 | 0,222 |
| 0,50 | 2,36 | 0,48 | 0,06 | - | - | 2,84 | 0,170 |
| 1,34 | 7,67 | 1,26 | 0,08 | - | - | 8,93 | 0,141 |
| 2,00 | 11,57 | 1,70 | 0,05 | - | - | 13,27 | 0,128 |
| 4,00 | 20,54 | 2,81 | 0,28 | 0,12 | - | 23,35 | 0,120 |
| 5,05 | 24,68 | 3,26 | 0,44 | 0,17 | - | 27,94 | 0,117 |
| 6,00 | 28,20 | 3,74 | 0,52 | 0,28 | - | 31,94 | 0,117 |
| 7,34 | 32,70 | 4,03 | 0,65 | 0,39 | - | 36,73 | 0,110 |

A partir des résultats obtenus dans les exemples 8 à 10 et dans les essais témoins (b) et (c) on a tracé les courbes ΣRR = f(t) (1) à (5) annexées pour chaque exemple (ou essai) en portant en abscisses le temps en heure et en ordonnées ΣRR en %.

La courbe (1) correspond à l'essai témoin (b) réalisé sans additif

La courbe (2) correspond à l'exemple 10

La courbe (3) correspond à l'exemple 8

La courbe (4) correspond à l'exemple 9

La courbe (5) correspond à l'essai témoin (c) réalisé avec un additif qui n'entre pas dans le cadre de l'invention.

## Revendications

1. Procédé de préparation du bisphénol-A par condensation du phénol en excès sur l'acétone en présence d'un catalyseur acide et d'un additif caractérisé en ce que l'additif répond à la formule (I) :

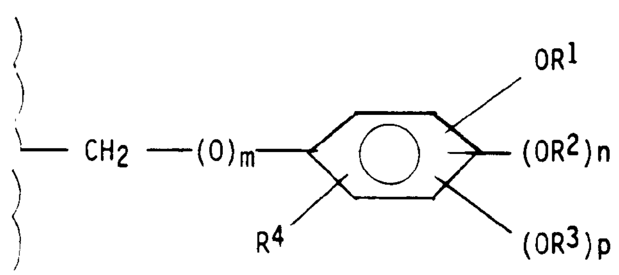

(I)

dans laquelle:

- m vaut 0 ou 1 ;
- n vaut 0 ou 1 ;
- p vaut 0 ou 1 ;
- $R^1$, $R^2$ et $R^3$, identiques ou différents, représentent un atome d'hydrogène, un radical alkyle en $C_1$-$C_6$ ou un radical phényle éventuellement substitué par 1 ou 2 groupements hydroxy ou alcoxy en $C_1$-$C_4$ ;

- $R^4$ représente un atome d'hydrogène, un atome d'halogène, un radical alkyle en $C_1$-$C_6$ ou l'un des groupements suivants :
  - CHO, - $NO_2$, - $CO_2H$, - $CO_2R^5$ où $R^5$ est un radical alkyle en $C_1$-$C_4$,

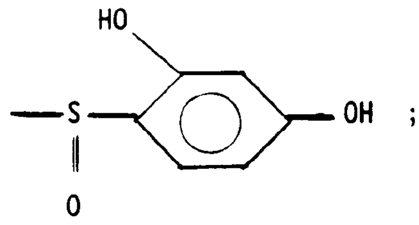

et,

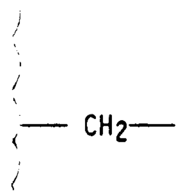

représente un reste de résine chlorométhylée.

2. Procédé selon la revendication 1 caractérisé en ce que la résine chloro-méthylée dont dérive l'additif de formule (I) est du type styrène-divinylbenzène.

3. Procédé selon la revendication 1 ou 2, caractérisé en ce que la résine chlorométhylée est choisie parmi les résines BIO-BEAD® S-X1, les résines DUOLITE® LES 9001 et DUOLITE® LES 3081.

4. Procédé selon l'une quelconque des revendications précédentes caractérisé en ce que au moins 90 % des atomes de chlore de la résine chlorométhylée sont remplacés par un groupe de formule (II)

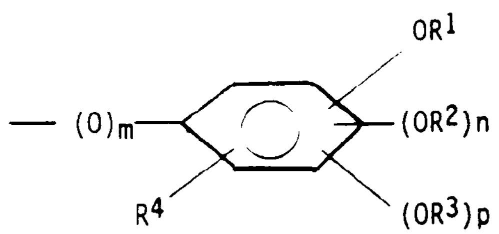

(II)

dans laquelle n, m, p, et $R^1$ à $R^4$ ont la signification donnée dans la revendication 1.

5. Procédé selon l'une quelconque des revendications précédentes caractérisé en ce que l'additif répond à la formule (I) dans laquelle m vaut 0.

6. Procédé selon l'une quelconque des revendications précédentes caractérisé en ce que l'additif répond à la formule (I) dans laquelle :
   - m est nul ;
   - $R^4$ représente un atome d'hydrogène ou un radical alkyle en $C_1$-$C_6$;
   - $R^1$, $R^2$ et $R^3$, identiques ou différents, représentent un atome d'hydrogène ou un radical alkyle en $C_1$-$C_4$.

7. Procédé selon l'une quelconque des revendications précédentes caractérisé en ce que l'additif répond à la formule (I) dans laquelle l'un au moins des radicaux $R^1$-$R^3$ représente un atome d'hydrogène et les deux autres, identiques ou différents, représentent un atome d'hydrogène ou un radical méthyle.

8. Procédé selon l'une quelconque des revendications précédentes caractérisé en ce que l'additif répond à la formule dans laquelle :
   - m est nul ;
   - p est nul ;
   - $R^4$ représente un atome d'hydrogène ou un radical alkyle en $C_1$-$C_6$ ;
   - les deux radicaux $R^1$ et $R^2$ représentent un atome d'hydrogène, les groupements hydroxyles ainsi présents sont en positions 2, 4 sur le noyau aromatique par rapport au groupement méthylène.

9. Procédé selon l'une quelconque des revendications précédentes caractérisé en ce que l'additif est,engagé à la réaction en quantité telle que le rapport molaire des groupes de formule II (donnée dans la revendication 4) à l'acétone soit compris entre 0,1 et 3.

10. Procédé selon l'une quelconque des revendications précédentes caractérisé en ce que le catalyseur acide est l'acide benzènesulfonique.

**Claims**

1. Process for the preparation of bisphenol A by condensation of excess phenol with acetone in the presence of an acid catalyst and of an additive, characterized in that the additive corresponds to the form (I):

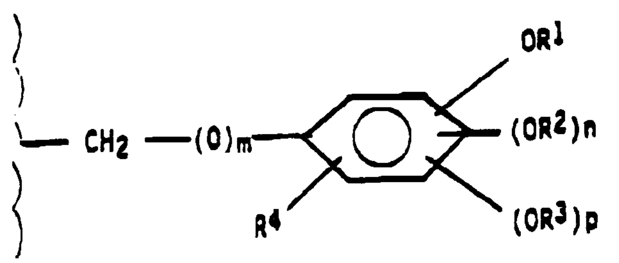

(I)

in which:
   - m has the value 0 or 1,
   - n has the value 0 or 1;
   - p has the value 0 or 1;
   - $R^1$, $R^2$ and $R^3$, which are identical or different, represent a hydrogen atom, a $C_1$-$C_6$ alkyl radical or a phenyl radical optionally substituted by 1 or 2 hydroxyl or $C_1$-$C_4$ alkoxy groups;
   - $R^4$ represent a hydrogen atom, a halogen atom, a $C_1$-$C_6$ alkyl radical or one of the following groups: -CHO, $-NO_2$, $-CO_2H$ or $-CO_2R^5$ where $R^5$ is a $C_1$-$C_4$ alkyl radical,

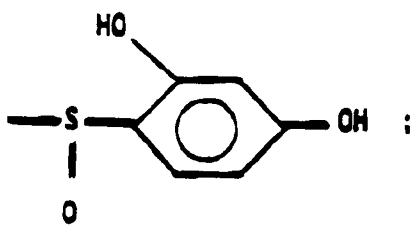

and,

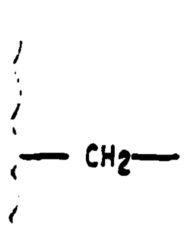

represents a chloromethylated resin residue.

2. Process according to claim 1, characterized in that the chloromethylated resin from which the additive of formula (I) derives is of the styrene-divinylbenzene type.

3. Process according to claim 1 or 2, characterized in that the chloromethylated resin is chosen from BIO-BEAD® S-XI resins or DUOLITE® LES 9001 and DUOLITE® LES 3081 resins.

4. Process according to any one of the preceding claims, characterized in that at least 90% of the chlorine atoms in the chloromethylated resin are replaced by a group of formula (II)

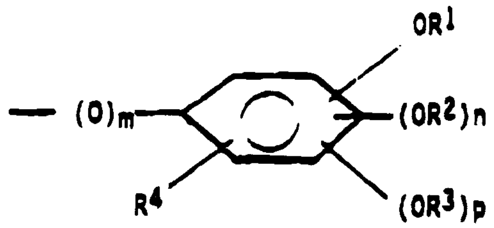

(II)

in which n, m, p and $R^1$ to $R^4$ have the meaning given in claim 1.

5. Process according to any one of the preceding claims, characterized in that the additive corresponds to the formula (I) in which m has the value 0.

6. Process according to any one of the preceding claims, characterized in that the additive corresponds to the formula (I) in which:
- m is 0;
- $R^4$ represents a hydrogen atom or a $C_1$-$C_6$ alkyl radical;
- $R^1$, $R^2$ and $R^3$, which are identical or different, represent a hydrogen atom or a $C_1$-$C_4$ alkyl radical.

7. Process according to any one of the preceding claims, characterized in that the additive corresponds to the formula (I) in which at least one of the $R^1$-$R^3$ radicals represents a hydrogen atom and the other two, which are identical or different, represent a hydrogen atom or a methyl radical.

8. Process according to any one of the preceding claims, characterized in that the additive corresponds to the formula in which:
- m is 0;
- p is 0;
- $R^4$ represents a hydrogen atom or a $C_1$-$C_6$ alkyl radical;
- the two $R^2$ and $R^2$ radicals represent a hydrogen atom and the hydroxyl groups thus present are in positions 2 and 4 on the aromatic ring with respect to the methylene group.

9. Process according to any one of the preceding claims, characterized in that the additive is charged to the reaction in an amount such that the molar ratio of the groups of formula II (given in claim 4) to the acetone is between 0.1 and 3.

10. Process according to any one of the preceding claims, characterized in that the acid catalyst is benzenesulphonic acid.

**Patentansprüche**

1. Verfahren zur Herstellung von Bisphenol-A durch Kondensation von Phenol im Überschuß mit Aceton, in Gegenwart eines sauren Katalysators und eines Additivs, dadurch gekennzeichnet, daß das Additiv der Formel (I)

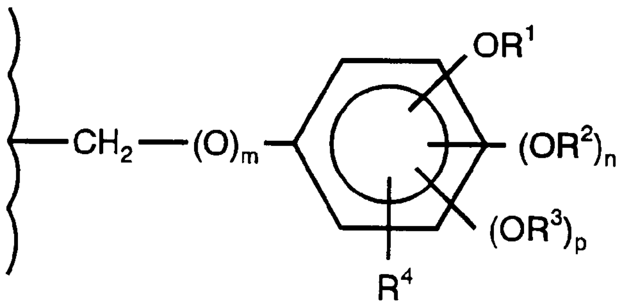

(I)

entspricht, in der:

- m 0 oder 1 ist;
- n 0 oder 1 ist;
- p 0 oder 1 ist;
- R1, R2 und R3, gleich oder verschieden, ein Wasserstoffatom, einen $C_1$-$C_6$-Alkylrest oder einen Phenylrest, der gegebenenfalls durch eine oder zwei Hydroxyl- oder $C_1$-$C_4$-Alkoxylgruppen substituiert ist, darstellen;
- $R_4$ ein Wasserstoffatom, ein Halogenatom, ein $C_1$-$C_6$-Alkylrest oder eine der folgenden Gruppen ist: -CHO, $-NO_2$, $-CO_2H$, $-CO_2R^5$, in der $R^5$ ein $C_1$-$C_4$-Alkylrest ist,

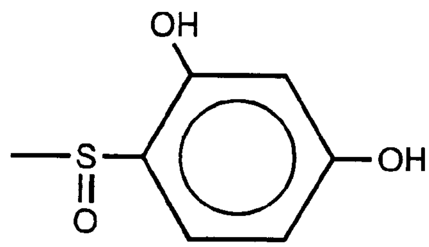

und

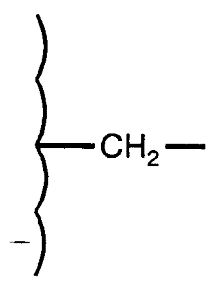

einen Rest eines chlormethylierten Harzes darstellt.

**2.** Verfahren nach Anspruch 1, dadurch gekennzeichnet, daß das chlormethylierte Harz, von dem das Additiv gemäß Formel (I) abgeleitet ist, vom Typ Styrol-Divinylbenzol ist.

**3.** Verfahren nach Anspruch 1 oder 2, dadurch gekennzeichnet, daß das chlormethylierte Harz ein Harz aus der Gruppe der BIO-BEAD® S-X1-, DUOLITE® LES 9001- und DUOLITE® LES 3081 ist.

**4.** Verfahren nach einem der vorhergehenden Ansprüche, dadurch gekennzeichnet, daß mindestens 90 % der Chloratome des chlormethylierten Harzes durch eine Gruppe der Formel (II)

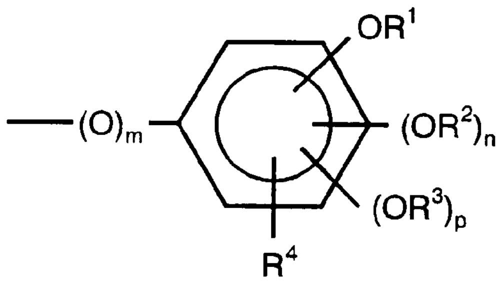

(II)

ersetzt sind, in der n, m, p, und $R^1$ bis $R^4$ die gleiche Bedeutung haben, wie in Anspruch 1.

**5.** Verfahren nach einem der vorhergehenden Ansprüche, dadurch gekennzeichnet, daß das Additiv der Formel (I) entspricht, in der m = 0 ist.

**6.** Verfahren nach einem der vorhergehenden Ansprüche, dadurch gekennzeichnet, daß das Additiv der Formel (I) entspricht, in der:
- m = 0 ist;
- $R^4$ ein Wasserstoffatom oder einen $C_1$-$C_6$-Alkylrest darstellt;
- $R^1$, $R^2$ und $R^3$, gleich oder verschieden, ein Wasserstoffatom oder einen $C_1$-$C_4$-Alkylrest darstellen.

**7.** Verfahren nach einem der vorhergehenden Ansprüche, dadurch gekennzeichnet, daß das Additiv der Formel (I) entspricht, in der mindestens einer der Reste $R^1$ bis $R^3$ ein Wasserstoffatom, und die beiden anderen, gleich oder verschieden, ein Wasserstoffatom oder einen Methylrest darstellen.

**8.** Verfahren nach einem der vorhergehenden Ansprüche, dadurch gekennzeichnet, daß das Additiv der Formel entspricht, in der
- m gleich 0 ist;
- p gleich 0 ist;
- $R^4$ ein Wasserstoffatom oder ein $C_1$-$C_6$-Alkylrest ist;
- die beiden Reste $R^1$ und $R^2$ ein Wasserstoffatom darstellen und in der sich die damit vorhandenen Hydroxylgruppen in den Positionen 2 und 4 zur Methylgruppe am aromatischen Ring befinden.

**9.** Verfahren nach einem der vorhergehenden Ansprüche, dadurch gekennzeichnet, daß das Additiv der Reaktion in einer solchen Menge zugegeben wird, daß das Molverhältnis der Gruppen gemäß Formel II (in Anspruch 4 angegeben) zu Aceton zwischen 0,1 und 3 liegt.

**10.** Verfahren nach einem der vorhergehenden Ansprüche. dadurch gekennzeichnet, daß der saure Katalysator Benzolsulfonsäure ist.

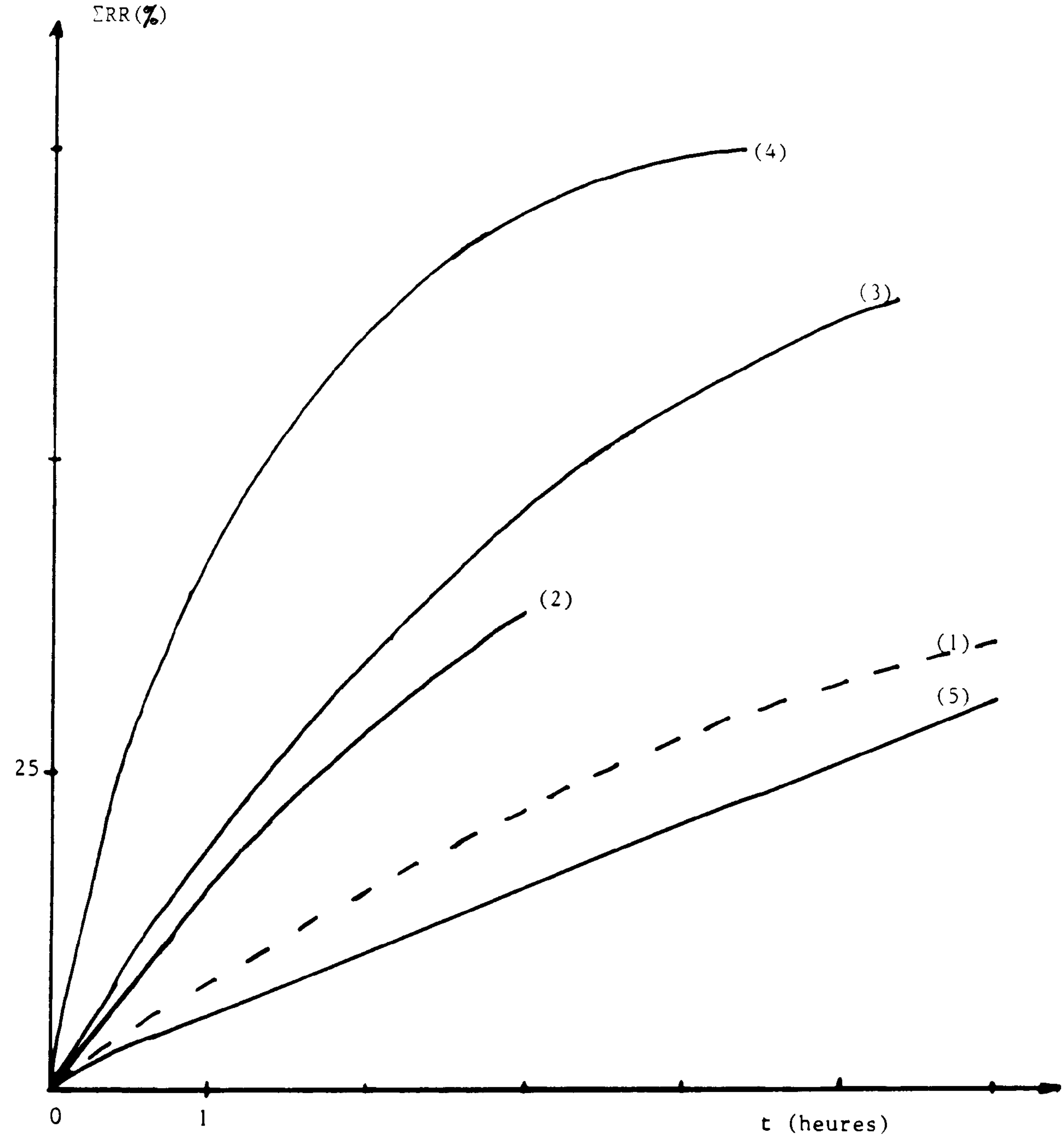

ΣRR(%)
(4)
(3)
(2)
(1)
(5)
25
0
1
t (heures)